(19) Europäisches Patentamt
European Patent Office
Office européen des brevets

(11) Veröffentlichungsnummer: **0 495 473 A1**

# (12) EUROPÄISCHE PATENTANMELDUNG

(21) Anmeldenummer: **92100563.3**

(22) Anmeldetag: **15.01.92**

(51) Int. Cl.5: **C07D 495/14**, A61K 31/55, //(C07D495/14,333:00,243:00, 233:00),(C07D495/14,333:00, 243:00,249:00)

(30) Priorität: **16.01.91 DE 4101146**

(43) Veröffentlichungstag der Anmeldung: **22.07.92 Patentblatt 92/30**

(84) Benannte Vertragsstaaten: **AT BE CH DE DK ES FR GB GR IT LI LU NL PT SE**

(71) Anmelder: **BOEHRINGER INGELHEIM KG**

**W-6507 Ingelheim am Rhein(DE)**

(84) **AT BE CH DE DK ES FR GB GR IT LI LU NL PT SE**

(71) Anmelder: **BOEHRINGER INGELHEIM INTERNATIONAL GmbH**
**Postfach 20**
**W-6507 Ingelheim am Rhein(DE)**

(84) **GB**

(72) Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Küfner-Mühl, Ulrike, Dr.**
**Schlossbergstrasse, 8**
**W-6507 Ingelheim/Rhein(DE)**
Erfinder: **Heuer, Hubert, Dr.**
**Am Sportfeld 74**
**W-6501 Schwabenheim(DE)**
Erfinder: **Birke, Franz, Dr.**
**Albrecht-Dürer-Strasse 23**
**W-6507 Ingelheim am Rhein(DE)**
Erfinder: **Bechtel, Wolf-Dietrich, Dr.**
**Mühlstrasse 3**
**W-6531 Appenheim(DE)**

(54) **Neue in 6-Position substituierte Diazepine, Verfahren zu ihrer Herstellung und ihre Verwendung als Arzneimittel.**

(57) Die Erfindung betrifft neue in 6-Position substituierte Diazepine, Verfahren zu ihrer Herstellung und ihre Verwendung als Arzneimittel

EP 0 495 473 A1

Die Erfindung betrifft neue Diazepine, Verfahren zu ihrer Herstellung sowie ihre Verwendung als Arzneimittel mit PAF-antagonistischer Wirkung

Die neuen Diazepine entsprechen der allgemeinen Formel I

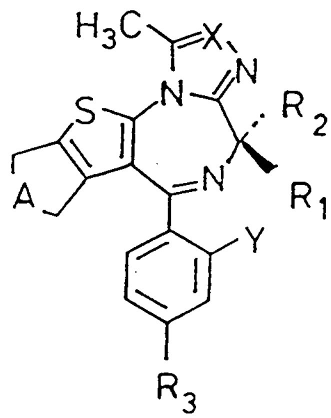

worin

A $-CH_2-$ oder $-CH_2-CH_2-$

X Stickstoff, C-H oder $C-CH_3$;

Y Wasserstoff oder Halogen;

$R_1$ Wasserstoff oder $CH_3$;

$R_2$ Wasserstoff oder $CH_3$;

$R_3$ 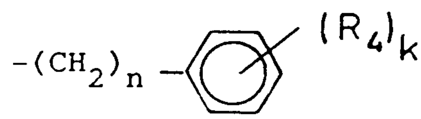 oder

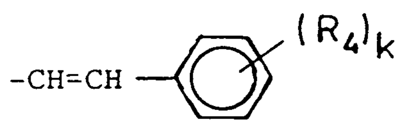 oder

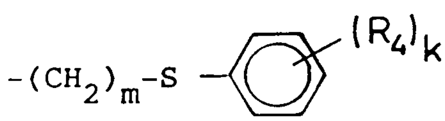 oder

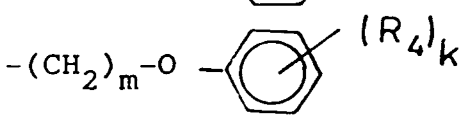

worin

n = 1, 2, 3 oder 4 - bevorzugt 2 - ,

m = 1, 2 oder 3 - bevorzugt 1 -,

$R_4$ Wasserstoff, Halogen, $CF_3$, $C_1$-$C_4$-Alkyl, Cycloalkyl, Methoxy, Trifluormethoxy, CN, k = 1, 2 oder 3 wobei bei k > 1 $R_4$ gleich oder verschieden sein kann,

bedeuten können,

mit der Maßgabe, daß wenn Y Wasserstoff bedeutet, $R_1$ und $R_2$ nicht beide zusammen Wasserstoff bedeuten können;

gegebenenfalls in Form ihrer Racemate, ihrer Enantiomeren, ihrer Diastereomere und ihrer Gemische.

Bevorzugt sind Diazepine der allgemeinen Formel Ia

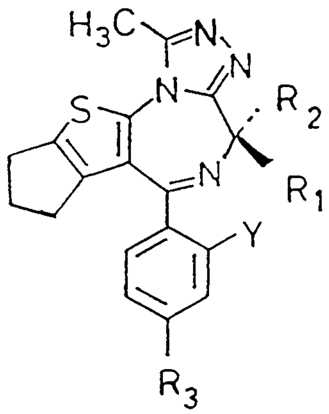

Ia

worin

Y Wasserstoff, Brom oder Chlor

$R_1$ und $R_2$ wie zuvor definiert sind;

$R_3$ 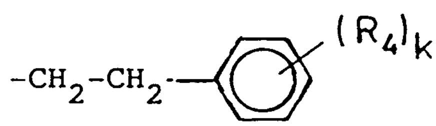 oder

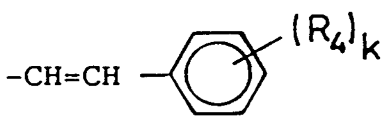 oder

(trans konfiguriert)

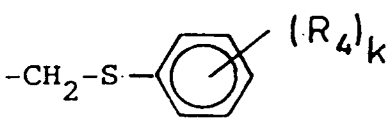 oder

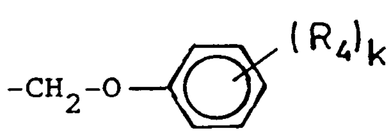

$R_4$ Wasserstoff, Chlor, Brom, Trifluormethyl, Methyl, iso-Butyl, Methoxy wobei $R_4$ bevorzugt in 4-Position des Phenylrings angeordnet ist, gegebenenfalls in Form ihrer Racemate, ihrer Entantiomere, ihrer Diastereomere und ihrer Gemische.

Die erfindungsgemäßen Verbindungen können im Diazepinring ein Asymmeriezentrum aufweisen, für den Fall, daß $R_1$ und $R_2$ verschieden sind.

Ganz besonders bevorzugt sind die S-konfigurierten Diazepine der allgemeinen Formel I und Ia, worin $R_1$ = Wasserstoff, $R_2$ = Methyl und Y = Halogen, bevorzugt Chlor, bedeuten.

Im Rahmen der Definitionen steht $C_1$ - $C_4$-Alkyl für eine verzweigte oder unverzweigte Alkylgruppe mit 1 bis 4 Kohlenstoffatomen, wie z.B. Methyl, Ethyl, Propyl, iso-Propyl, n-Butyl, iso-Butyl, sek.-Butyl und tert.-Butyl. Unter der Definition Halogen wird F, Cl, Br oder Jod verstanden.

Die bei der Synthese gegebenenfalls anfallenden Gemische optisch isomerer Verbindungen können durch Bildung von Diastereomeren und nachfolgender an sich bekannte Verfahren, wie z.B. durch Kristallisation, durch chromatographische oder enzymatische Trennverfahren in die einzelnen optischen Isomeren getrennt werden.

Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile
und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin. PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (z. B. akute und chronische Bronchitis, Asthma bronchiale) oder der Niere (z. B. Glomerulonephritis) oder der Gelenke (z. B. rheumatische Erkrankungen): anaphylaktische Zustände, Allergien und Entzündungen im Bereich der Schleimhäute (z.B. allergische Rhinitis) und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni; zur Behandlung von Thrombosen.
Weiterhin erscheinen die erfindungsgemäßen Verbindungen zur Behandlung bei den folgenden Indikationsstellungen geeignet:
Obstruktive Lungenerkrankungen, bronchiale Hyperreaktivität; entzündliche Lungenwegserkrankungen, wie z.B. chronische Bronchitis; fibrotische Lungenwegserkrankungen, wie z.B. Fibrosing alveolitis und interstital pneumonitis syndrome; gynokologische Erkankungen z.B. Dysmennorhea, Schwangerschaftseklampsie, Schwangerschaftsbluthochdruck; Hemmung der Geburtswehen; Herz- Kreislauferkrankungen, wie z.B. Polytrauma, Anaphylaxie, Arteriosklerose; Krankheiten der fehlerhaften Blutgefäßbildung, z.B. fehlerhafte Blutgefäßbildung in den Augen; EPH-Gestose (edema-proteinuria Hypertension); entzündliche und immunologische Erkrankungen; Immunmodulation bei Transplantationen von Fremdgeweben, Immunmodulation bei Leukämie, Metastasenausbreitung z.B. bei bronchialer Neoplasie; weiterhin erweisen sich die erfindungsgemäßen Verbindungen als Cyto- und Organoprotektiv, z.B. zur Neuroprotektion, z.B. bei Leberzirrhose, z.B. Schutz bei Strahlenbehandlung, z.B. Anwendung während oder nach
chirurgischen Eingriffen und in extra-Korporal Kreisläufen; DIC (disseminierte intravasale Gerinnung); zum Einsatz bei Organtransplantationen, zur Verminderung der Nebenwirkungen einer Arzneimitteltherapie, z.B. anaphylaktoide Kreislaufreaktionen, Kontrastmittelzwischenfälle, Nebenwirkungen bei der Tumortherapie; haemolytik uremic syndrome; hepta-renal syndrome, Unverträglichkeiten bei Bluttransfusionen; Leberversagen (z.B. Hepatitis, primär biliar zirrhosis, primär sclerosende cholangitis), Vergiftungen, z.B. Amanitaphalloides-Intoxikation
[Knollenblätterpilzvergiftung];
Symptome von parasitären Erkrankungen (z.B. Wurmerkrankungen); Autoimmunerkrankungen.
Weiterhin sind folgende Indikationen von Interesse: Immunfunktion bei Aids, Diabetes, juvenile Diabetes, diabetische Retinopathie, polytraumatischer Schock, hämorrhagischer Schock, Erkrankungen des ZNS, z.B. Migräne, Agoraphobie (panic disorder): Ischämie, Multiple Sklerose; entzündliche Darmerkrankungen; Colitis Ulcerosa, Morbus Crohn: pulmonaler Hochdruck sowie chronische ischämische Herzinsuffizienz, PAF Antagonisten der allgemeinen Formel I eignen sich zur Behandlung von pathologischen Blutgasveränderungen, wie beispielsweise respiratorische Acidose, metabolische Alkalose. Allein oder in Kombination mit Anticholinergica können PAF-Antagonisten zur Verbesserung der Blutgaswerte bei Phosphorsäureestervergiftungen eingesetzt werden. Es ist bekannt, daß PAF-Antagonisten allein - oder in Kombination mit immunsuppressiv wirkenden Verbindungen (z.B. Cyclosporine oder FK-506) zur Behandlung von Asthma, Autoimmunerkrankungen und bei Transplantationen eingesetzt werden können, wobei nicht nur eine Verbesserung der Wirksamkeit aber auch eine Verminderung der Toxizität von Cyclosporinen oder FK-506 zu erwarten sind. Weiterhin wird vorgeschlagen, PAF-Antagonisten in Kombination mit Antihistaminica zu verwenden. Bezüglich der Definition der Antihistaminica wird inhaltlich auf die Europäische Patentanmeldung 345 731 Bezug genommen. Ferner ist bekannt, daß PAF-Antagonisten in Kombination mit $\beta_2$-Mimetica zur Behandlung des Asthma bronchiale eingesetzt werden können.
PAF assoziierte Interaktion mit Gewebshormon (autocoid hormones), Lymphokine und anderen Mediatoren sind bekannt. Auch die Kombination von PAF-Antagonisten mit TNF sind vorteilhaft.

Wässerige Lösungen, die einen der erfindungsgemäßen Verbindungen enthalten, eignen sich zur

Aufbewahrung von Organen, die zur Transplantation bestimmt sind. PAF-Antagonisten eignen sich als Zusatz zu Blut- und Plasma-Konserven.

Die neuen Hetrazepine sind sehr starke PAF-Antagonisten und anderen bekannten diazepinoiden PAF-Antagonisten in folgenden Kriterien überlegen:

- es besteht eine totale Dissoziation zwischen dem PAF-Antagonismus und den Benzodiazpin-Receptor vermittelten Effekten;
- überlegende Bindungsaffinität zum PAF-Rezeptor auf gewaschenen Humanplättchen und Zeigen eine stärkere Hemmung der PAF-induzierten Plättchenaggregation;
- Darüber hinaus hemmen sie in einer überlegenen Weise die PAF-(30 ng/kg x min) induzierte Bronchokonstriktion nach oraler und parenteraler Applikation am Meerschweinchen in Kombination mit einer sehr langen Wirkdauer (größer 15 Std. nach oraler Applikation am Meerschweinchen).

Die Hemmung der PAF-induzierten Plättchenaggregation kann nach folgender Methodik bestimmt werden.

Gesunden männlichen und weiblichen Spendern im Alter von 18 bis 35 Jahren, die mehrere Tage vor Blutentnahme keine Medikamente (Aspirin oder andere nicht steroidale Entzündungshemmer) eingenommen hatten, wurden jeweils 200 ml Blut aus einer nicht gestauten Vene mit Hilfe einer Plastikspritze, in der sich 3.8 %-ige Natriumcitratlösung befand, entnommen. Das Verhältnis Natriumcitratlösung: Blut betrug 1:9. Das Citratblut wurde in Plastikröhrchen bei 150 x g ( = 1200 U/min) und Raumtemperatur 20 min lang zentrifugiert (Heraeus Christ Tischzentrifuge 124).

Die Messung der Thrombozytenaggregation in vitro erfolgte nach der Methode von Born und Cross (1963), wobei dem TRP unter ständigem Rühren ein Aggregationsauslöser (PAF) zugesetzt wurde. Zur Messung wurde in 1 ml Plastikküvetten, die jeweils einen kleinen Metallstift (Rührer, 1000 U/min) enthielten, 0.8 ml TRP und 0.2 ml modifizierte Tyrode-Lösung (s. unten) gegeben. Die Prüfsubstanz wurde 2 bis 3 min vor Auslösung der Aggregation in einem Volumen von 10 $\mu$l zugesetzt. Als Lösungsmittel dienten entweder DMSO und Wasser oder eine verdünnte HCl-Lösung. Die Kontrollansätze enthielten das entsprechende Volumen dieser Lösungsmittel. Nach Registrierung der Ausgangsabsorption (2-3 min) wurde die Aggregation induziert. In einem Volumen von 10 $\mu$l erfolgte die Gabe von PAF (5 x $10^{-8}$ M; Bachem Feinchemikalien) in die Küvette.

Die modifizierte Tyrode-Lösung hatte folgende Zusammensetzung: 136.9 mM NaCl; 2.68 mM KCl; 0.5 mM $MgCl_2$; 1.8 mM $CaCl_2$; 0.42 mM $NaH_2PO_4$; 5.55 mM Glucose und 11.9 mM $NaHCO_3$.

Zur Beurteilung von Substanzeffekten wurde das Maximum der ersten Aggregationswelle verwendet. Die durch den Aggregationsauslöser induzierte maximale Absorption (= maximale Aggregation = 100 %) wurde gleichzeitig in einem Parallelansatz (im 2. Kanal des Aggregometers) zu jedem Testansatz mitgeführt und als 100 % Wert verwendet. Der unter der Wirkung der Testsubstanz erreichte Aggregationswert wurde als % des Kontrollwertes (-ansatz) angegeben. Mit Hilfe dieses Verfahrens wurden Konzentrationswirkungskurven mit einem Stichprobenumfang von jeweils n = 4 erstellt und $IK_{50}$-Werte (Konzentration bei einer 50 %igen Aggregationshemmung) berechnet.

Die Herstellung der erfindungsgemäßen Verbindungen kann in Analogieverfahren gemäß dem Stand der Technik erfolgen.

In Anlehnung an die Europäische Patentschrift 194 416 und die Europäischen Patentanmeldungen 230 942 und 254 245 erfolgt die Synthese gemäß nachfolgenden Raktionen.

Ausgehend von dem entsprechend substituierten Diazepinthionen der allgemeinen Formel

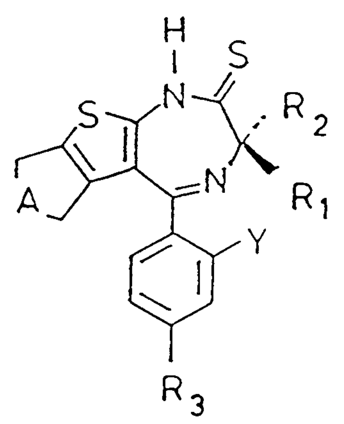

worin A, $R_1$, $R_2$, $R_3$ und $R_4$ wie zuvor definiert sind, erhält man die erfindungsgemäßen Verbindungen der allgemeinen Formel I bzw. Ia dadurch, daß man

A) für den Fall, daß X Stickstoff bedeutet

a) mit einem Säurehydrazid der allgemeinen Formel $CH_3$-CONHNH$_2$ (III) umsetzt,

b) oder aber mit Hydrazin in das entsprechende Hydrazon überführt und anschließend mit einem Säurehalogenid der allgemeinen Formel $CH_3$-CO-Hal oder mit einem Orthoester der allgemeinen Formel $CH_3$-C(OR')$_3$ worin R' eine niedere Alkylgruppe bedeutet, umsetzt, oder

B) Für den Fall, daß X C-H,

a) mit einem Aminoalkin der allgemeinen Formel $R_1$'- C≡C - $CH_2$-$NH_2$ worin $R'_1$ Wasserstoff bedeutet oder aber

b) mit einem α-Aminoaldehyd-alkylacetal oder α-Aminoketon-alkylketal der allgemeinen Formel $H_2NCH_2$-$CCH_3$(OR')$_2$ worin R' eine niedere Alkylgruppe bedeutet, umsetzt und anschließend gegebenenfalls die so erhaltenen Verbindungen mittels an sich bekannter Trennverfahren in ihre optisch aktiven Verbindungen spaltet.

Die Umsetzung des Thions II mit einem Säurehydrazid III nach dem Verfahren a) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen der Raumtemperatur und dem Siedepunkt des Reaktionsgemisches. Die Isolierung der Endprodukte geschieht nach bekannten Methoden, wie z.B. durch Kristallisation.

Die Umsetzung des Thions II mit Hyrazin nach dem Verfahren b) erfolgt in inerten organischen Lösungsmitteln, wie beispielsweise Tetrahydrofuran, Dioxan, halogenierten Kohlenwasserstofen, wie z.B. Methylenchlorid, geeigneten Kohlenwasserstoffen, bei Temperaturen zwischen Raumtemperaturen und dem Siedepunkt des Reaktionsgmisches.

Die dabei entstehenden Hydrazin-1,4-diazepine können nach herkömmlichen Verfahren isoliert oder aber auch direkt weiterverarbeitet werden.

Die weitere Umsetzung mit einem Säurehalogenid oder Orthoester erfolgt in einem inerten organischen Lösungsmittel, wie z.B. halogenierten Kohlenwasserstoffen, cyclischen oder aliphatischen Estern, kann aber auch direkt in Substanz erfolgen. Die Isolierung des Endprodukts Ia erfolgt nach bekannten Methoden, beispielsweise durch Kristallisation. Weitere Einzelheiten des Herstellverfahrens sind dem ausführlichen Reaktionsschema sowie den Beispielen zu entnehmen.

Verbindungen der allgemeinen Formel

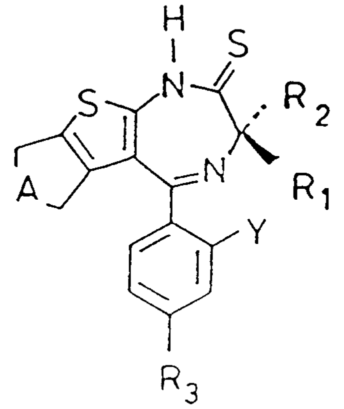

worin A $R_1$, $R_2$, $R_3$ und $R_4$ wie zuvor definiert sind, sind wertvolle Ausgangsprodukte zur Herstellung von Hetrazepinen mit PAF-antagonistischer Wirkung und werden als solche beansprucht.

Der Aufbau des Grundgerüstes der erfindungsgemäßen Verbindungen erfolgt auf an sich bekannte Weise [EP-OS 254 245 und EP-PS 194 416] und ist dem folgenden Reaktionsschema retrosynthetisch skizziert; jedoch ohne das Verfahren auf die gezeigten Reste einzuschränken.

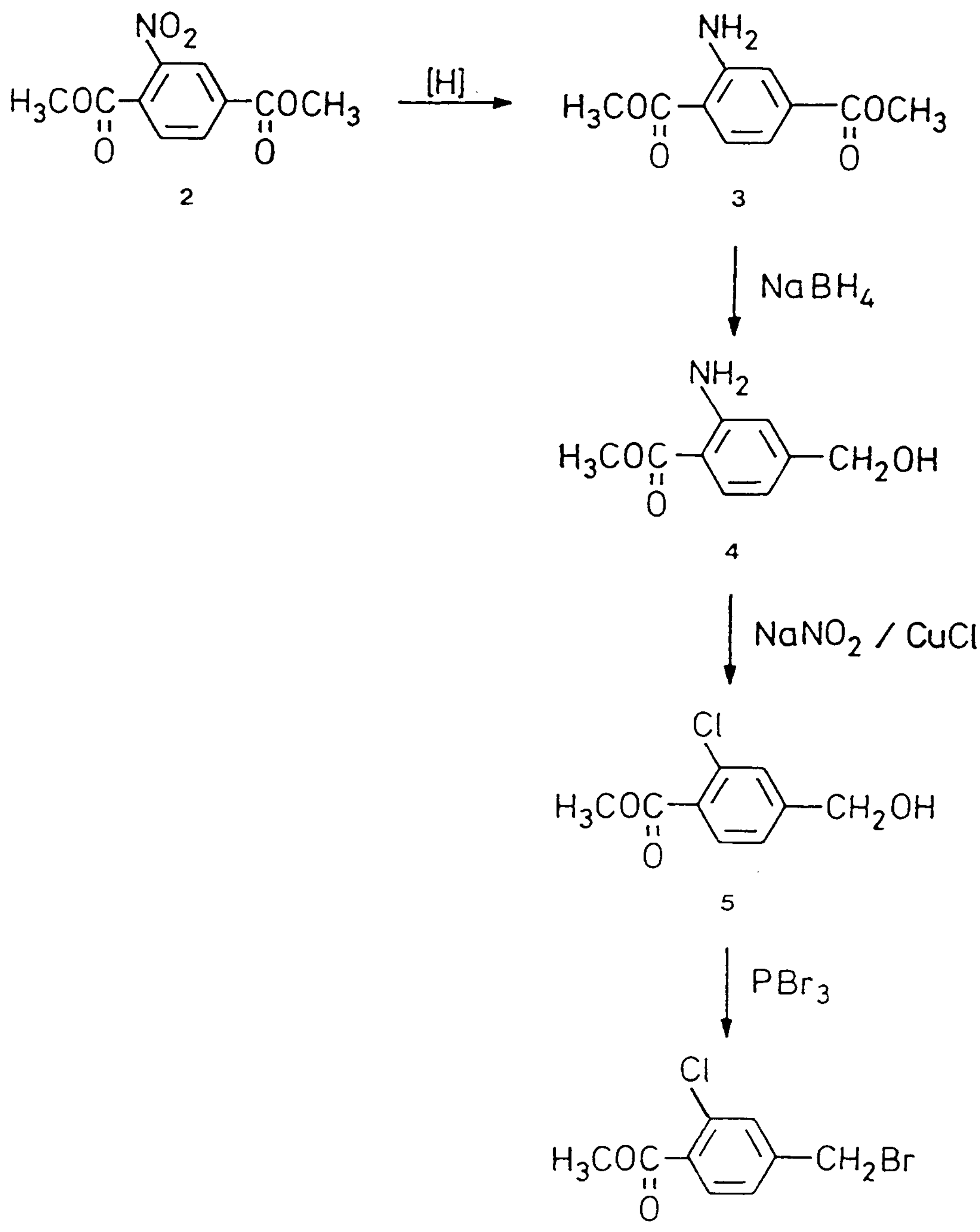


2

3

4

5

6

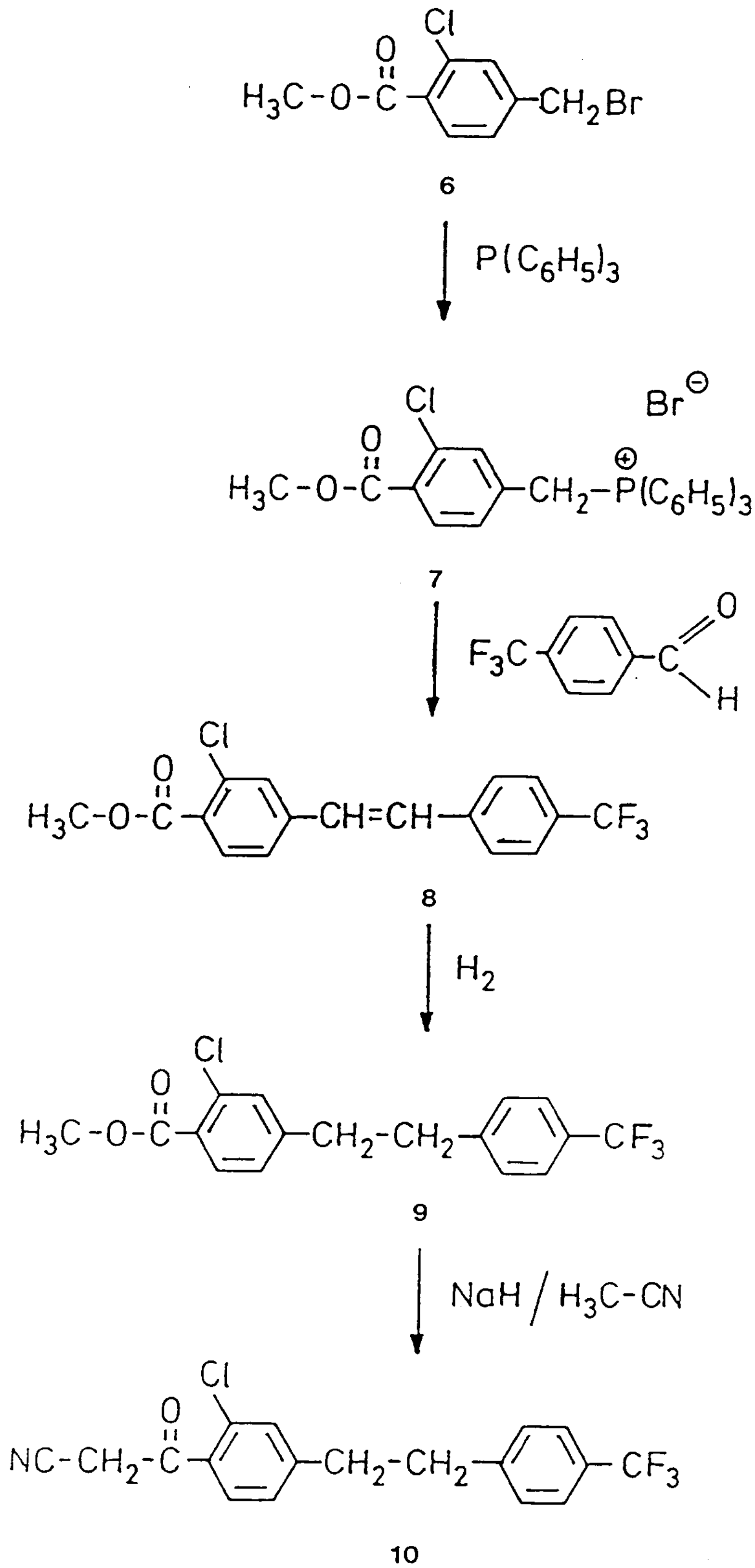


6

7

8

9

10

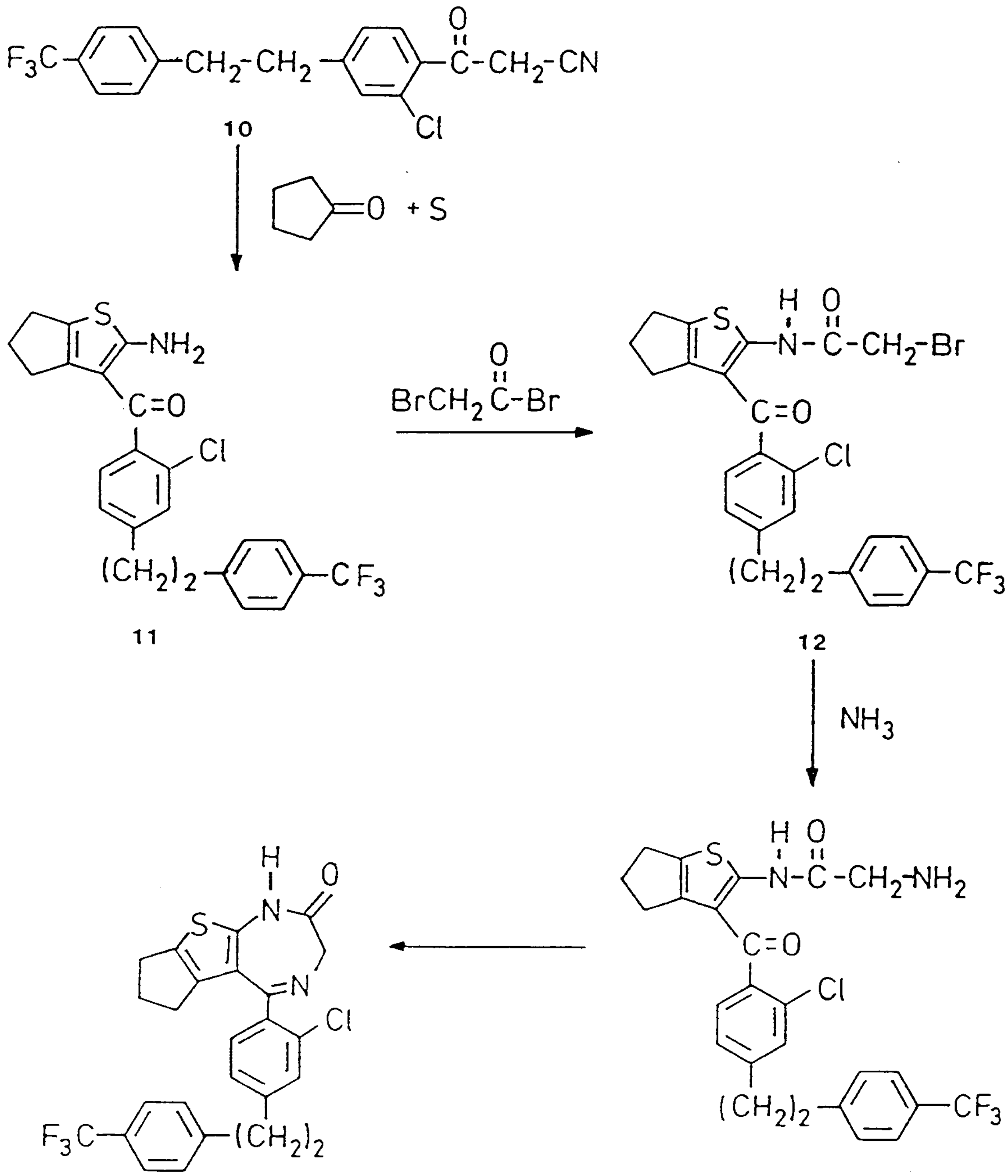


10

11

12

13

14

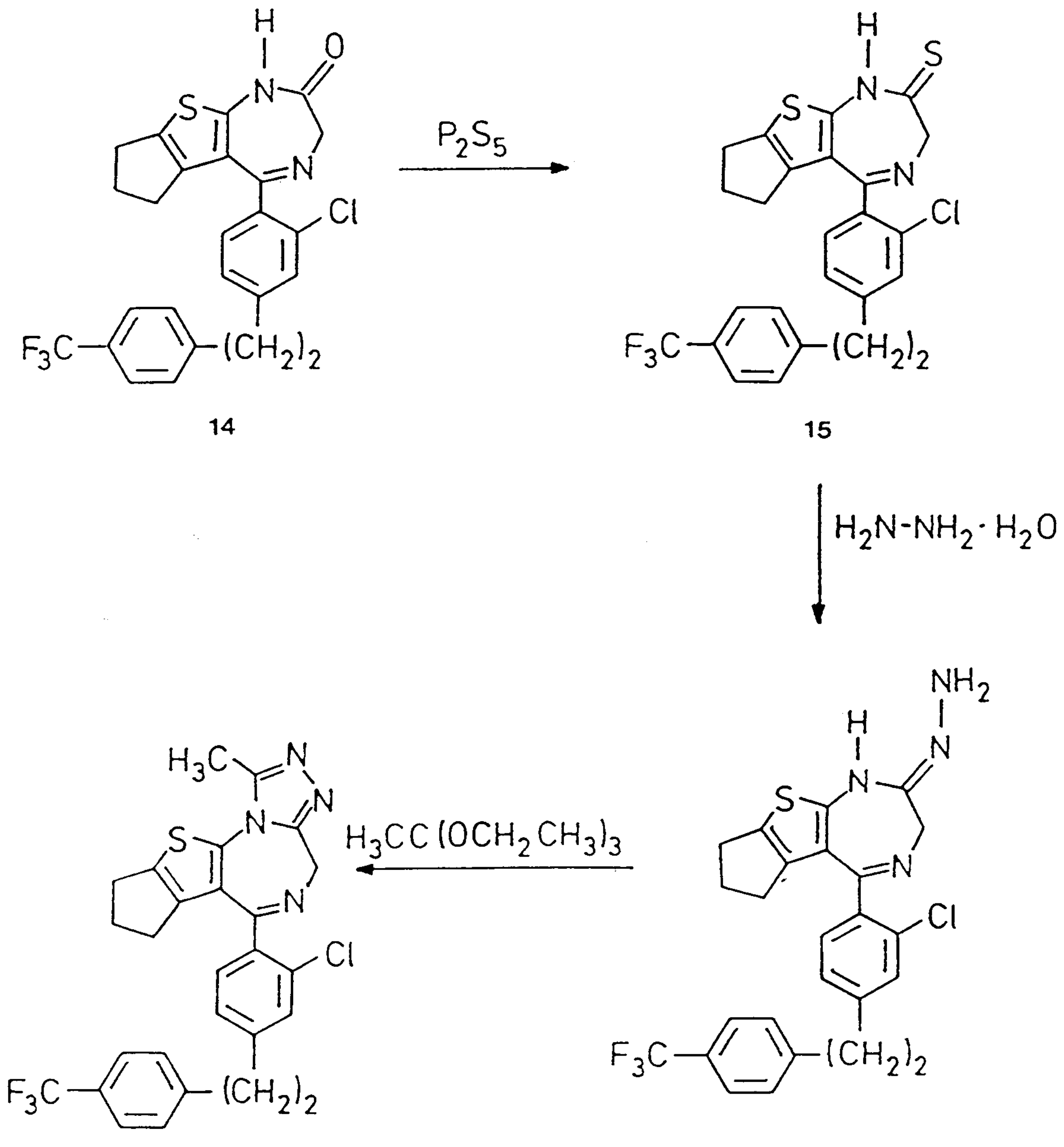


14

15

17

16

Beispiel 1

6(2-Chlor-4-[2-C-(4-trifluormethylphenyl)ethyl]phenyl)-8,9-dihydro-1-methyl-4H,7H-cyclopenta[4,5]thieno[3,2-f]-[1,2,4]triazolo[4,3-a][1,4]-diazepin

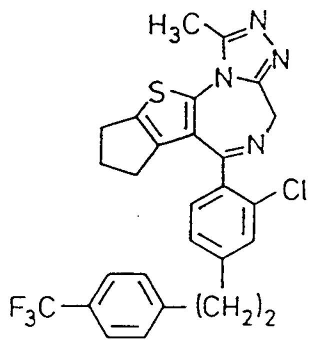

a) 2-Aminotherephthalsäuredimethylester

23,9 g (0,1 Mol) 2-Nitrotherephthalsäuredimethylester werden in einem Gemisch von 100 ml Methanol und 200 ml Tetrahydrofuran mit Palladiumkohle bei 5 bar und 20 ° C hydriert. Nach Absaugen des Katalysators dampft man das Lösungsmittel ab und kristallisiert den Rückstand aus Methanol um. Ausbeute 18 - 19 g, gelbliche Kristalle vom Fp. 130 - 131 ° C.

b) 2-Amino-4-hydroxymethyl-benzoesäuremethylester

10,5 g (0,05 Mol) 2-Aminotherephthalsäuredimethylester werden in 150 ml tert. Butanol suspendiert und nach Zugabe von 5,7 g 0,15 Mol Natriumborhydrid auf 85 ° erwärmt. Nach einstündigem Rühren bei dieser Temperatur werden unter gleichzeitigem Rückflußkochen allmählich 15 ml Methanol zugetropft. Anschließend kocht man noch 1 Stunde unter Rühren, kühlt das Reaktionsgemisch ab und schüttelt dreimal mit 50 ml gesättigter Kochsalzlösung aus. Das Butanol wird im Vakuum abdestilliert und der Rückstand aus Essigester umkristallisiert. Man erhält 6 - 7 g des gewünschten Aminoalkohols als weiße Kristalle vom Fp. 102 - 103 ° C, die nur wenig des isomeren Carbinols enthalten.

$^1$H-NMR ($CD_3OD$):$\delta$ = 7.72(1H,d,J = 7.0Hz,); 6.73(1H,d,J = 2Hz,); 6.52(1H,dd,J = 7.0, 2.0Hz,); 4.50 (2H,s,$CH_2$-O); 3.80 (3H,s,$OCH_3$); $NH_2$, OH in the solvent blind peak.

c) 2-Chlor-4-hydroxymethyl-benzoesäuremethylester

18,1 g (0,1 Mol) obiger Aminoverbindung werden in 50 ml Wasser und 50 ml konz. Salzsäure gelöst. Bei 2 - 5 ° C fügt man unter Rühren allmählich eine Lösung von 6,9 Natriumnitrit in 50 ml Wasser hinzu und rührt noch 5 Minuten nach. Die erhaltene Diazoniumsalzlösung wird nun bei 5 ° allmählich zu 14 g Kupfer-I-chlorid in 100 ml konzentrierter Salzsäure getropft. Anschließend wird das Reaktionsgemisch 15 Minuten auf 85 ° erwärmt. Man kühlt ab, schüttelt zweimal mit 100 ml Methylenchlorid aus, trocknet die organische Phase, und destilliert das Lösungsmittel ab. Es hinterbleiben 16 - 17 g farbloses Carbinol.

$^1$H-NMR($CD_3OD$):$\delta$ = 7.77(1H,d,J = 7.0Hz,); 7.47(1H,d,J = 2.0Hz, Hm); 7.32(1H,dd,J = 7.0, 2.OHz,); 4.62(2H,s,$CH_2$-O); 3.89(3H,s,$OCH_3$); OH in the solvent blind peak.

d) 2-Chlor-1-carbomethoxy-triphenylphosphoniummethylbromid

34 g (0,17 Mol) obigen Carbinols werden in 400 ml Methylenchlorid gelöst und bei 5 - 8 ° C langsam unter Rühren mit 10 ml Phosphortribromid versetzt. Man rührt 1 Stunde bei Raumtemperatur nach, verschiebt den pH-Wert durch Zugabe von verdünntem Ammoniak auf 8, trennt die Methylenchloridphase ab und erhält nach Trocknen und Eindampfen 26 - 28 g Rückstand vom Fp. 39 - 40 ° C. Diesen nimmt man in 200 ml Benzol auf, fügt 26,5 g Triphenylphosphin hinzu, und kocht 6 Stunden unter Rückfluß. Nach Abkühlen werden die Kristalle abgesaugt und mit wenig Benzol gewaschen. Man erhält nach dem Trocknen 50 - 55 g reines Triphenylphosphoniumsalz vom Fp. 226 - 228 ° C.

e) 2-Chlor-4-trifluormethylphenylethylenbenzoesäuremethylester

4 g Natriumhydriddispersion (50 %ig) werden zu 100 ml absolutem Dimethylsulfoxid gegeben und unter Stickstoff 45 Minuten bei 80 ° C gerührt. Man kühlt auf 10 ° C ab und fügt unter weiterer Kühlung 53 g des obigen Phosphoniumsalzes portionsweise hinzu. Nach etwa 10 Minuten bildet sich eine Lösung, die bei Raumtemperatur mit 17,4 g 4-Trifluorbenzaldehyd versetzt wird. Man rührt 2 Stunden bei Raumtemperatur, verdünnt mit 3000 ml Essigester und schüttelt das Gemisch zweimal mit je 100 ml Wasser aus. Aus der organischen Phase erhält man 26 g des erwarteten cis-trans-Isomerengemisches, das sich duch Behandlung mit Diisopropyläther auftrennen läßt. Ausbeute: 8 g einer kristallinen Fraktion der trans-Verbindung vom Fp. 90 - 92 ° C und 20 g der öligen cis-Verbindung.

f) 2-Chlor-4-trifluormethylethylbenzoesäuremethylester

18 g obiges Olefin werden in 300 ml Tetrahydrofuran unter Zuhilfenahme von Raney-Nickel bei 5 bar und 20 ° C hydriert. Nach Abtrennen des Katalysators, Eindampfen des Lösungsmittels und Chromatographie des Rückstandes über $SiO_2$ (Laufmittel Toluol) erhält man 17 g des gewünschten Esters vom Fp. 58 - 59 ° C.

| | | | | | | |
|---|---|---|---|---|---|---|
| ber.<br>gef. | C | 59.57,<br>59.90, | H | 4.12,<br>4.45 | Cl | 10.34<br>9.98 |

g) 2-Chlor-4-trifluormethylphenylethylphenylcarbonylacetonitril

Eine Mischung von 17 g (0,25 Mol) obigen Esters, 240 ml Toluol, 4 ml Acetonitril und 2,4 g Natriumhydriddispersion (50 %ig) werden unter kräftigem Rühren 6 Stunden unter Rückfluß gekocht. Nach dem Abkühlen fügt man 250 ml Essigester hinzu und säuert das Gemisch langsam mit 2 n Salzsäure an. Die Wasserphase wird abgetrennt, die organische Phase nochmals mit Wasser gewaschen und getrocknet. Nach dem Eindampfen und Chromatographieren über $SiO_2$ (Laufmittel Toluol) erhält man 12 g des gewünschten Cyanoketons neben 5 g Ausgangsmaterial.

h) 2-Amino-3-(2-chlor-4(-ethyl-4-(trifluormethylphenyl)) benzoyl-cyclopentano[4,5]tiophen

12 g obiges Cyanoketon, 30 ml Ethanol, 3 g Cyclopentanon, 1,1 g Schwefel und 2,8ml Triethylamin werden unter Rückfluß 6 Stunden gekocht. Man dampft das Reaktionsgemisch im Vakuum ein, verdünnt den Rückstand mit Methylenchlorid, wäscht mit Wasser, trocknet, destilliert das Lösunsmittel erneut ab und chromatographiert den Rückstand über $SiO_2$ (Laufmittel Methylenchlorid/Methanol (99 : 1)).

Man erhält 6 - 8 g des gewünschten Aminothiophens. Analog dem in unserer Anmeldung EP 254 245 beschriebenen Verfahren wird ausgehend von obigem Aminothiophen durch Bromacylierung, gefolgt von einer Behandlung mit gasförmigem Ammoniak in Essigester und Nachbehandlung der Aminoverbindung mit $SiO_2$ in siedendem Toluol am Wasserabscheider das entsprechende 1,4-Diazpepinon aufgebaut.

Aus diesem erhält man durch Behandlung mit Phosphorpentasulfid das Diazepinthion und hieraus die entsprechende Hyrazinoverbindung vom Fp. 198 - 200 ° C. Letztere wird wie bereits in der Europäischen Patentanmeldung 254 245 beschrieben mit Ortho-essigsäure- triethylester in die gewünschte Titelverbindung überführt, die bei 200 - 201 ° C schmilzt.

$^1$H-NMR($CDCl_3$):$\delta$ = 7.53-7.02(7H,m,aryl-H);
4.86(2H,s,broad, $CH_2$-7ring);
2.96(6H,m,aryl-$CH_2CH_2$; $CH_2$-9; 5ring);
2.70(3H,s,$CH_3$-triazole); 2.38-1.82(4H,m,$CH_2$8/7;
5ring).

Beispiel 2

6-[4-[2-(4-Trifluormethylphenyl)ethyl]phenyl]-8,9-dihydro-1,4,4-trimethyl-4H,7H-cyclopenta[4,5]thieno-[3,2-f]-[1,2,4]triazolo[4,3-a][1,4]diazepin

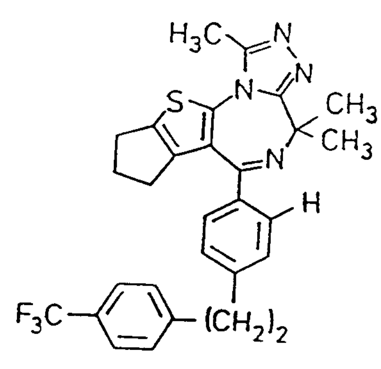

a) 11,5 g (70 mmol) 4-Hydroxymethylbenzoesäuremethylester werden in 120 ml Dichlormethan gegeben und mit 47,3 ml Phosphortribromid versetzt. Man rührt eine Stunde bei Raumtempertur und gibt unter Eiskühlung vorsichtig halbkonzentrierte Lösung von Ammoniak in Wasser hinzu bis ein pH-Wert von 8 bis 9 erreicht ist. Die organische Phase wird abgetrennt, getrocknet und das Lösungsmittel abdestilliert.

Man erhält 13,4 g (84 % d. Th.) des 4-Brommethylbenzoesäuremethylesters (in Analogie zu Verbindung 6).

b) Phosphoniumsalz:

13,3 g (58 mmol) des Brommethylbenzoesäuremethylesters werden mit 15,1 g Triphenylphosphin in 130 ml Benzol 6 Stunden unter Rückfluß gekocht. Die bei der Reaktion ausgefallenen Kristalle werden abgesaugt und getrocknet. Man erhält 27,4 g (96 % d. Th.) des Phosphoniumbromids vom Typ 7 als Kristalle vom Fp. 258-260 °C.

c) Wittig-Olefinierung

In einer Stickstoff-Atmosphäre werden 8,7 g 55 %-ige Natriumhydrid-Dispersion in 100 ml absolutes Dimethylsulfoxid gegeben und 45 Minuten bei 80 °C gerührt. Nach Abkühlen auf Raumtemperatur tropft man eine Suspension von 98 g (0,2 mol) des Phosphoniumsalzes 4 ein und rührt das Reaktionsgemisch 10 Minuten nach. Darauf werden 34,8 g Trifluormethylbenzaldehyd eingetropft und 2 Stunden bei Raumtemperatur gerührt. Anschließend wird das Gemisch mit 400 ml Essigester verdünnt und 2 mal mit Wasser gewaschen. Die organische Phase wird getrocknet und der Rückstand chromatographiert ($SiO_2$-Säule, Elutionsmittel: Dichlormethan). Die Hauptfraktion wird im Vakuum eingedampft und der Rückstand aus Isopropylether umkristallisiert. Man erhält 37,5 g (61 % d. Th.) des Stilbens vom Typ 8 als Kristalle vom Fp. 157-158 °C

d) 37 g (0,12 mol) des so gewonnenen Olefins werden in 600 ml Tetrahydrofuran mit Raney-Nickel als Katalysator bei 20 °C und 5 bar hydriert. Nach Absaugen des Katalysators dampft man das Tetrahydrofuran ab und erhält 33,5 g des entsprechenden Diphenylethanderivats vom Typ 9 in Form weißer Kristalle (90 % d. Th.) vom Fp. 88-90 °C.

e) 33 g (0,1 mol) des so hydrierten Esters werden in 100 ml Toluol gelöst. Man fügt 4,8 g einer 55 %-igen Natriumhydrid-Dispersion und anschließend 6,9 ml Acetonitril hinzu und kocht das Reaktionsgemisch 6 Stunden unter Rückfluß. Nach dem Abkühlen wird mit verdünnter Salzsäure auf pH 5 bis 6 angesäuert. Die Suspension wird 3 mal mit Dichlormethan extrahiert, die organischen Extrakte getrocknet und im Vakuum eingedampft. Den Rückstand chromatographiert man über eine $SiO_2$-Säule mit Dichlormethan als Elutionsmittel. Die Hauptfraktion wird eingedampft, wobei der Rückstand kristallisiert. Ausbeute: 15,5 g (46 % d. Th.); Fp.: 105 °C.

f) 155 g (488 mmol) des Cyanoketons vom Typ 10, 46 g Cyclopentanon und 17,4 g Schwefel werden in 270 ml Dimethylformamid suspendiert bzw. gelöst. Man fügt anschließend 38 ml Triethylamin zu und rührt 3 Stunden bei 60 °C nach. Nach dem Abkühlen wird das Reaktionsgemisch mit Essigester verdünnt und die organische Phase 2 mal mit Wasser gewaschen. Nach Abdampfen der Lösungsmittel im Vakuum wird der Rückstand auf eine $SiO_2$-Säule gegeben und das gewünschte Produkt mit Dichlormethan eluiert. Der Rückstand läßt sich durch Zugabe von Isopropylether/Petrolether kristallisieren. Man erhält 64 g des Aminoketons vom Typ 11 (32 % d. Th.) mit einem Fp. von 140-150 °C.

g) 20,7 g (0,05 Mol) des so hergestellten 2-Amino-3-[[2-(4-trifluormethylphenyl)ethyl]phenyl]-benzoylcyclopentano[4,5]thiophens werden in 200 ml Dichlormethan gelöst bzw. suspendiert und mit 5,3 ml Pyridin versetzt. Unter Rühren tropft man bei Raumtemperatur 6,8 ml (0,055 Mol) 2-Brom-isobuttersäurebromid hinzu und rührt 1 Stunde nach. Das Reaktionsgemisch wird im Vakuum auf 1/3 des Volumens eingedampft und anschließend über eine $SiO_2$-Säule gegeben. Man eluiert mit Dichlormethan und erhält aus dem Rückstand nach dem Einengen des Eluats durch Umkristallisieren aus einer Mischung Äther/Petroläther 22,5 g der Bromverbindung vom Typ 12 in Form hellgelber Kristalle vom Fp. 132 - 135 °C.

h) 11,3 g der Bromverbindung vom Typ 12 werden mit 14 ml Essigester, 10 ml Dichlorethan und 1 g flüssigem Ammoniak versetzt und 1 Stunde bei 100 °C und 8 bar geschüttelt. Das erkaltete Reaktionsgemisch wird mit Wasser gewaschen. Aus der organischen Phase werden nach dem Trocknen und Eindampfen 10 g der entsprechenden Aminoverbindung vom Typ 13 in Form eines zähflüssigen Öles erhalten.

i) Die so dargestellte Aminoverbindung wird zusammen mit 50 g $SiO_2$ und 200 ml Toluol 2 Stunden am Wasserabscheider unter Rühren auf Rückflußtemperatur erhitzt. Nach dem Erkalten saugt man das Kieselgel ab und extrahiert dreimal mit je 100 ml Methanol. Der Rückstand der vereinigten und eingeengten Eluate wird an einer $SiO_2$-Säule chromatographiert (Dichlormethan/Methanol 99 : 1). Man erhält 4 g des gewünschten Diazepinons vom Typ 14. Hieraus werden gemäß der EP 254 245 mit Phosphorpentasulfid in Pyridin bei 60 °C das entsprechende Thion vom Typ 15 als rotes Festprodukt erhalten, das mit Hydrazinhydrat in das entsprechende Hydrazid vom Typ 16 überführt wird. 1,75 g dieses Hydrazids werden in 10 ml Ethanol und 1,5 ml Orthoessigsäuretriethylester aufgenommen und 1 Stunde unter Rückfluß gekocht. Man dampft das Reaktionsgemisch im Vakuum ein, chromatographiert an einer mit $SiO_2$ gefüllten Säule (Elutionsmittel: Dichlormethan/Methanol 97 : 3) und erhält aus der

Hauptfraktion nach der Zugabe von Diethylether die Titelverbindung als Kristalle vom Fp. 187 - 189 ° C.
$^1$H-NMR($CDCL_3$):δ = 7.52-7.03(8H,m,aryl-H);
2.95(6H,m,aryl-$CH_2CH_2$; $CH_2$-9, 5ring);
2.69(3H,s,$CH_3$-triazole);
2.37-1.95(4H,m,$CH_2CH_2$ 8/7); 1.19(6H,s,broaden, $C(CH_3)_2$

Beispiel 3

6-(4-[2-(4-trifluormethylphenyl)ethyl]phenyl)-8,9-dihydro-1-methyl-R,S-4-methyl-4H,7H-cyclopenta [4,5]-thieno[3,2-f][1,2,4]triazolo[4,3-a[1,4]diazepin

Ausgehend von 2-Amino-3(4-ethyltrifluormethylphenyl)-benzoylcyclopentano[4,5]thiophen (vgl. EP 254 245) wurde mit 2-Brompropionsäurebromid analog vorstehender Methode das entsprechende Propionylderivat erhalten, das mit Ammoniak in Essigester/Dichloräthan und Nachbehandlung mit $SiO_2$ in siedendem Toluol das entsprechende racemische Diazepinon ergibt. Mit Phospentasulfid erhält man hieraus das Thion als gelbe Kristalle vom Fp. 220 ° C. Die hieraus zugängliche Hydrazinoverbindung schmilzt bei 223 ° C und ergibt mit Ortho-Essigsäuretriethylester die racemische Titelverbindung vom Schmelzpunkt 162 - 163 ° C.
$^1$H-NMR($CDCL_3$):δ = 7.51-7.03(8H,m,aryl-H);
4.20(1H,qu,J = 6.0Hz, CH-$CH_3$);
2.95(6H,m,aryl-$CH_2CH_2$; $CH_2$-9, 5ring);
2.62(3H,s,$CH_3$-triazole); 2.43-2.02(4H,m,$CH_2$ 8/7 5ring); 2.11(3H,d,J = 6Hz, CH-$CH_3$).

Enantiomerentrennung mit Hilfe einer chiralen Säule

1 g des oben erhaltenen Racemates werden in 10 ml einer Mischung von Cyclohexan/Isopropanol 60 : 40 im Ultraschallbad gelöst und auf eine Chiraspher-Säule der Firma E. Merck, Darmstadt (Korngröße 5 µm) aufgegeben. Mit einem Fluß von 2 ml/Minute wird mit Cyclohexan/Isopropanol 60 : 40 eluiert. Dies geschieht im Recycling-Betrieb. Nach vollständiger Trennung der beiden Enantiomeren werden diese Lösungen präparativ aufgearbeitet.

Man erhält das Eutomere (das pharmakologisch wirksame Enantiomere) (S-Konfiguration) als zweite Fraktion nach dem Abdestillieren des Elutionsmittel als Kristalle vom Fp. 164 - 165 ° und einem Drehwert $[\alpha]_D$ = + 49,9 (Methanol) und einer optischen Reinheit von größer 99 %.

Die erste Fraktion beinhaltet die distomere (das pharmakologisch weniger wirksame Enantiomere) Verbindung (R-Konfiguration) mit einem Drehwert von $[\alpha]_D$ -48 ° (Methanol).

In Analogie zu dem allgemeinen Syntheseschema und den ausführlich beschriebenen Beispielen lassen sich - beispielsweise - folgende Verbindungen herstellen :

6-{2-Chlor-4-[2-(4-trifluormethylphenyl)ethyl]phenyl}-8,9-dihydro-1,4-dimethyl-4H,7H-cyclopenta[4,5]thieno-[3,2-f]-[1,2,4]triazolo[4,3-a][1,4]diazepin

6-{2-Chlor-4-[2-(4-trifluormethylphenyl)ethyl]phenyl}-8,9-dihydro-1-methyl-4H,7H-cyclopenta[4,5]thieno[3,2-f][1,2,4]-triazolo[4,3-a][1,4]diazepin

6-{4-[2-(4-Trifluormethylphenyl)ethyl]phenyl}-8,9-dihydro-1,4-dimethyl-4H,7H-cyclopenta[4,5]thieno[3,2-f]-[1,2,4]triazolo-[4,3-a][1,4]diazepin

6-[2-Chlor-4-(2-phenylethyl)phenyl]-8,9-dihydro-1,4-dimethyl-4H,7H-cyclopenta[4,5]thieno[3,2-f]-[1,2,4]-triazolo[4,3-a][1,4]-diazepin

6-[2-Chlor-4-(2-phenylethyl)phenyl]-8,9-dihydro-1-methyl-4H,7H-cyclopenta[4,5]thieno[3,2-f]-[1,2,4]triazolo-[4,3-a][1,4]diazepin

6-[4-(2-Phenylethyl)phenyl]-8,9-dihydro-1,4-dimethyl-4H,7H-cyclopenta[4,5]thieno[3,2-f][1,2,4]triazolo[4,3-a]-[1,4]diazepin

6-{2-Chlor-4-[2-(4-chlorphenyl)ethyl]phenyl}-8,9-dihydro-1,4-dimethyl-4H,7H-cyclopenta[4,5]thieno[3,2-f]-[1,2,4]triazolo-[4,3-a][1,4]diazepin

6-{2-Chlor-4-[2-(4-chlorphenyl)ethyl]phenyl}-8,9-dihydro-1-methyl-4H,7H-cyclopenta[4,5]thieno[3,2-f][1,2,4]-triazolo[4,3-a]-[1,4]diazepin

6-{4-[2-(4-Chlorphenyl)ethyl]phenyl}-8,9-dihydro-1,4-dimethyl-4H,7H-cyclopenta[4,5]thieno[3,2-f][1,2,4]-triazolo[4,3-a][1,4]-diazepin

6-{2-Chlor-4-[(4-trifluormethylphenyl)thiomethyl]phenyl}-8,9-dihydro-1,4-dimethyl-4H,7H-cyclopenta[4,5]-thieno[3,2-f][1,2,4]triazolo[4,3-a][1,4]diazepin

6-{2-Chlor-4-[(4-trifluormethylphenyl)thiomethyl]phenyl}-8,9-dihydro-1-methyl-4H,7H-cyclopenta[4,5]thieno-[3,2f][1,2,4]triazolo[4,3-a][1,4]diazepin

6-{4-[(4-Trifluormethylphenyl)thiomethyl]phenyl}-8,9-dihydro-1,4-dimethyl-4H,7H-cyclopenta[4,5]thieno[3,2-f]-[1,2,4]triazolo[4,3-a][1,4]diazepin
(4S)-6-{2-Chlor-4-[2-(4-trifluormethylphenyl)ethyl]phenyl}-8,9-dihydro-1,4-dimethyl-4H,7H-cyclopenta[4,5]-thieno[3,2-f][1,2,4]triazolo[4,3-a][1,4]diazepin
(4S)-6-{4-[2-(4-Trifluormethylphenyl)ethyl]phenyl}-8,9-dihydro-1,4-dimethyl-4H,7H-cyclopenta[4,5]thieno-[3,2f][1,2,4]triazolo[4,3-a][1,4]diazepin
(4S)-6-[2-Chlor-4-(2-phenylethyl)phenyl]-8,9-dihydro-1,4-dimethyl-4H,7H-cyclopenta[4,5]thieno[3,2f][1,2,4]-triazolo[4,3-a][1,4]diazepin
(4S)-6-[4-(2-Phenylethyl)phenyl]-8,9-dihydro-1,4-dimethyl-4H,7H-cyclopenta[4,5]thieno[3,2-f][1,2,4]triazolo-[4,3-a][1,4]diazepin
(4S)-6-{2-Chlor-4-[2-(4-chlorphenyl)ethyl]phenyl}-8,9-dihydro-1,4-dimethyl-4H,7H-cyclopenta[4,5]thieno[3,2-f][1,2,4]triazolo[4,3-a][1,4]diazepin
(4S)-6-{4-[2-(4-Chlorphenyl)ethyl]phenyl}-8,9-dihydro-1,4-dimethyl-4H,7H-cyclopenta[4,5]thieno[3,2-f][1,2,4]-triazolo[4,3-a][1,4]diazepin
(4S)-6-{2-Chlor-4-[(4-trifluormethylphenyl)thiomethyl]phenyl}-8,9-dihydro-1,4-dimethyl-4H,7H-cyclopenta-[4,5]thieno[3,2-f][1,2,4]triazolo[4,3-a][1,4]diazepin
(4S)-6-[4-[-Trifluormethylphenyl)thiomethyl]phenyl]-8,9-dihydro-1,4-dimethyl-4H,7H-cyclopenta[4,5]thieno-[3,2-f][1,2,4]triazolo[4,3-a][1,4]diazepin

Galenische Zubereitungen

Beispiel a

Tabletten, enthaltend 10 mg Substanz B

Zusammensetzung:

| | |
|---|---|
| Substanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockne Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg
Substanz B = 6(2-Chlor-4-ethylphenyl-(4-trifluormethylphenyl))-8,9-dihydro-1-methyl-4H,7H-cyclopenta[4,5]thieno[3,2-f][1,2,4]triazolo[4,3-a][1,4]diazepin

Beispiel b

Dragées, enthaltend 5 mg Substanz B

| Zusammensetzung: | |
|---|---|
| Substanz B | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 ° C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel c

Tabletten, enthaltend 50 mg Substanz B

| Zusammensetzung: | |
|---|---|
| Substanz B | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:
Die Substanz B, Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50 ° C getrocknet und erneut gesiebt. Nach Zumischen des Schmiermittels wird das Granulat auf einer Tablettiermaschine gepreßt.

Beispiel d

Kapseln, enthaltend 50 mg Substanz B

| Zusammensetzung: | |
|---|---|
| Substanz B | 50,0 mg |
| Maisstärke | 268,5 mg |
| Magnesiumstearat | 1,5 mg |
| | 320,0 mg |

Herstellung:
Substanz B und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

Beispiel e

Suppositorien, enthaltend 50 mg Substanz B

| Zusammensetzung: | |
|---|---|
| Substanz B | 50 mg |
| Adeps solidus | 1.650 mg |
| | 1.700 mg |

Herstellung:
Das Hartfett wird geschmolzen. Bei 40 ° C wird die gemahlene Wirksubstanz homogen dispergiert. Es wird auf 38 ° C abgekühlt und in schwach vorgekühlte Suppositorienformen ausgegossen.

Beispiel f

Orale Suspension, enthaltend 50 mg Substanz B pro 5 ml

| Zusammensetzung: | |
|---|---|
| Substanz B | 50 mg |
| Hydroxyethylcellulose | 50 mg |
| Sorbinsäure | 5 mg |
| Sorbit 70%ig | 600 mg |
| Glycerin | 200 mg |
| Aroma | 15 mg |
| Wasser ad | 5 ml |

Herstellung:
Destilliertes Wasser wird auf 70 ° C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz B zugegeben. Zur Entlüftung der Suspension wird unter Rühren evakuiert.

Selbstverständlich lassen sich die übrigen erfindundungsgemäßen PAF-Antagonisten in den für sie geeigneten Dosierungen in die üblichen galenischen Zubereitungen einarbeiten.

Eine wirksame Dosis der erfindungsgemäßen Verbindungen liegt bei oraler Anwendung zwischen 1 und 200, vorzugsweise zwischen 20 und 60 mg/Dosis, bei intravenöser oder intramuskulärer Anwendung zwischen 0,001 und 50, vorzugsweise zwischen 0,1 und 20 mg/Dosis. Für die Inhalation sollen Lösungen, die 0,01 bis 1,0, vorzugsweise 0,1 bis 1 % Wirkstoff enthalten, eingesetzt werden.

**Patentansprüche**

**1.** Neue Diazepine der allgemeinen Formel I

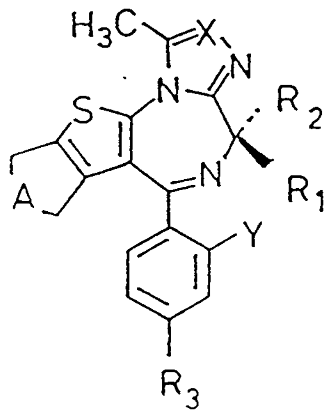

worin

A $-CH_2-$ oder $-CH_2-CH_2-$

X Stickstoff, C-H oder $C-CH_3$;

Y Wasserstoff oder Halogen;

$R_1$ Wasserstoff oder $CH_3$;

$R_2$ Wasserstoff oder $CH_3$;

$R_3$ 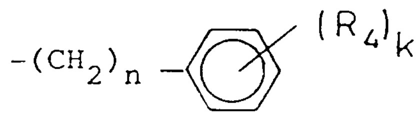 oder

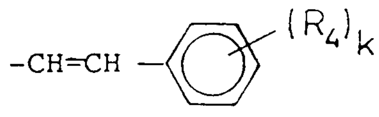 oder

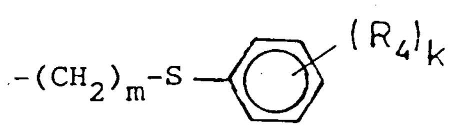 oder

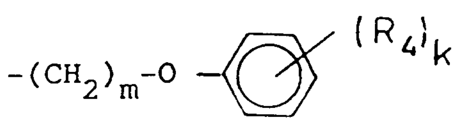

worin

n = 1, 2, 3 oder 4 - bevorzugt 2 - ,

m = 1, 2 oder 3 - bevorzugt 1 -,

$R_4$ Wasserstoff, Halogen, $CF_3$, $C_1$-$C_4$-Alkyl, $C_3$ - $C_6$ Cycloalkyl, Methoxy, Trifluormethoxy, CN,

k = 1, 2 oder 3 wobei bei k > 1 $R_4$ gleich oder verschieden sein kann,

bedeuten können,

mit der Maßgabe, daß wenn Y Wasserstoff bedeutet, $R_1$ und $R_2$ nicht beide zusammen Wasserstoff bedeuten können;

gegebenenfalls in Form ihrer Racemate, ihrer Enantiomeren, ihrer Diastereomere und ihrer

Gemische.

**2.** Neue Diazepine der allgemeinen Formel Ia

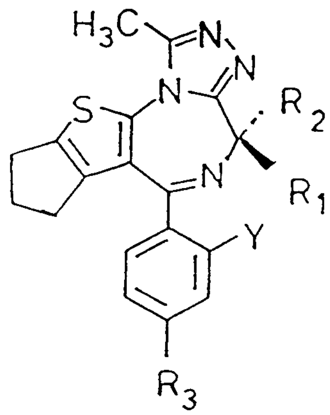

Ia

worin

Y Wasserstoff, Brom oder Chlor

$R_1$ und $R_2$ wie zuvor definiert sind;

$R_3$ $-CH_2-CH_2$ 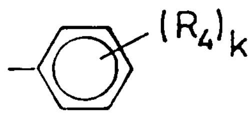 oder

$-CH=CH$ 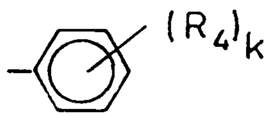 oder

(trans konfiguriert)

$-CH_2-S$ 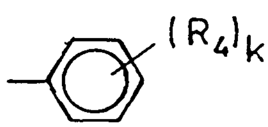

oder

$-CH_2-O$ 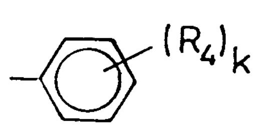

$R_4$ Wasserstoff, Chlor, Brom, Trifluormethyl, Methyl, iso-Butyl, Methoxy wobei $R_4$ bevorzugt in 4-Position des Phenylrings angeordnet ist, gegebenenfalls in Form ihrer Racemate, ihrer Entantiomere, ihrer Diastereomere und ihrer Gemische.

**3.** S-konfigurierte Diazepine der allgemeinen Formel Ia nach Anspruch 2, worin Y Halogen, $R_1$ Wasserstoff und $R_2$ Methyl bedeuten.

4. Pharmazeutische Zubereitungen, gekennzeichnet durch einen Gehalt an einer Verbindung nach einem der Ansprüche 1 bis 3.

5. Verwendung von Verbindungen nach einem der Ansprüche 1 bis 3 bei der Herstellung von pharmazeutischen Zubereitungen zur Behandlung von Erkrankungen, die durch endogen gebildetes PAF induziert werden.

6. Verwendung von Verbindungen nach einem der Ansprüche 1 bis 3 als Arzneimittel.

7. Verbindungen der allgemeinen Formel

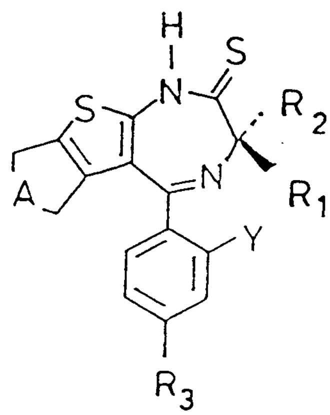

worin A, $R_1$, $R_2$, $R_3$ und $R_4$ wie in einem der vorhergehenden Ansprüche definiert sind.

8. Verfahren zur Herstellung von Verbindungen der allgemeinen Formel

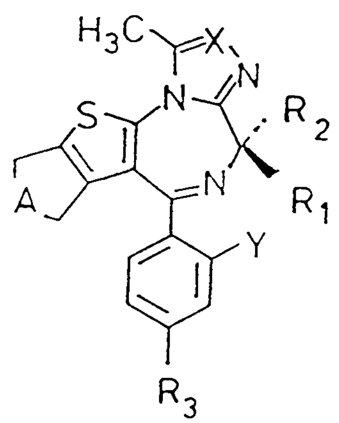

worin A, $R_1$, $R_2$, $R_3$ und $R_4$ wie in einem der vorhergehenden Ansprüche definiert, dadurch gekennzeichnet, daß entsprechend substituierte Diazepinthione der allgemeinen Formel

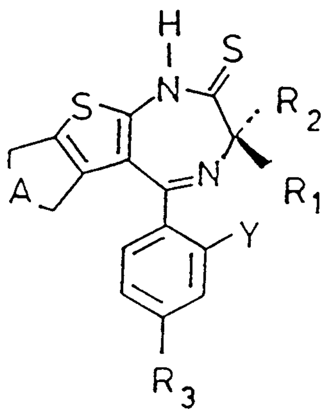

worin A, $R_1$, $R_2$, $R_3$ und $R_4$ wie zuvor definiert sind,

A) für den Fall, daß X Stickstoff bedeutet

a) mit einem Säurehydrazid der allgemeinen Formel $CH_3$-CONHNH$_2$ (III) umsetzt,

b) oder aber mit Hydrazin in eine Verbindung der allgemeinen Formel überführt und anschließend mit einem Säurehalogenid der allgemeinen Formel $CH_3$-CO-Hal oder mit einem Orthoester der allgemeinen Formel $CH_3$-C(OR')$_3$ worin R' eine niedere Alkylgruppe bedeutet, umsetzt, oder

B) für den Fall, daß X C-H,

a) mit einem Aminoalkin der allgemeinen Formel

$R_1$'- C≡C - $CH_2$-$NH_2$

worin $R'_1$ Wasserstoff

bedeutet

oder aber

b) mit einem α-Aminoaldehyd-alkylacetal oder α-Aminoketon-alkylketal der allgemeinen Formel

$H_2NCH_2$-$CCH_3$(OR')$_2$

worin R' eine niedere Alkylgruppe bedeutet, umsetzt und anschließend gegebenenfalls die so erhaltenen Verbindungen mittels an sich bekannter Trennverfahren in ihre optisch aktiven Verbindungen spaltet.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 92100563.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A - 0 407 955 (BOEHRINGER INGELHEIM KG) * Ansprüche 1,4-6,10 * | 1,2, 4-6,7, 8 | C 07 D 495/14<br>A 61 K 31/55<br>//(C 07 D 495/14<br>C 07 D 333:00<br>C 07 D 243:00<br>C 07 D 233:00)<br>(C 07 D 495/14<br>C 07 D 333:00<br>C 07 D 243:00<br>C 07 D 249:00) |
| A | DE - A - 3 909 012 (BOEHRINGER INGELHEIM KG) * Ansprüche 1,2,8 * | 1,2, 4-6,7, 8 | |
| A | DE - A - 3 724 031 (BOEHRINGER INGELHEIM KG) * Ansprüche 1,2,5,8-11 * | 1,2, 4-6,7, 8 | |

RECHERCHIERTE SACHGEBIETE (Int Cl 5)

C 07 D 495/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7-8

Unvollständig recherchierte Patentansprüche: -

Nicht recherchierte Patentansprüche: 6

Grund für die Beschränkung der Recherche:

(Verfahren zur therapeutischen Behandlung des menschlichen oder tierischen Körpers, EPÜ Art. 52(4)

| Recherchenort | Abschlußdatum der Recherche | Prüfer |
|---|---|---|
| WIEN | 16-03-1992 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument